# FASCICULE DE BREVET EUROPEEN

(11) **EP 1 749 494 B1**
(45) Date de publication et mention de la délivrance du brevet: **05.12.2012**
(21) Numéro de dépôt: 06300855.1
(22) Date de dépôt: 03.08.2006
(51) Int. Cl.: A61B 18/20

(54) **Appareil de traitement par émission de flashs lumineux, notamment d'épilation**
Gerät zur Behandlung mit Lichtblitzen, speziell zur Epilation
Apparatus for treatment with light flashes, especially for depilation

(30) Priorité: 05.08.2005 FR 0552452
(43) Date de publication de la demande: 07.02.2007
(73) Titulaire: Eurofeedback, 91017 Evry Cedex (FR)
(72) Inventeur: Safraoui, Georges, 91140, Villejust (FR)
(74) Mandataire: Tanty, François

(56) Documents cités:
- US-A1- 2004 068 255
- US-A1- 2005 137 655

## Description

La présente invention concerne les appareils de traitement par émission de flashs lumineux, notamment d'épilation, de photorajeunissement, et/ou d'élimination de défauts cutanés ou vasculaires.

Un appareil de traitement par émission de flashs lumineux est décrit dans la demande de brevet français FR 2 838 042.

Un tel appareil comporte un poste de base et une pièce à main comportant au moins une lampe flash et reliée par un câble au poste de base.

La pièce à main est décrite de manière détaillée dans la demande de brevet français FR 2 864 440.

Selon le traitement effectué, par exemple le phototype, l'énergie envoyée par le générateur électrique du poste de base vers la lampe flash doit être différente.

Il en résulte que la lampe flash peut présenter une durée de vie qui varie en fonction de l'utilisation qui en est faite. Au cours de son vieillissement, la lampe flash perd en efficacité et une énergie électrique plus grande doit être envoyée dans celle-ci pour un résultat équivalent.

Dans les appareils connus, une opération de maintenance périodique est ainsi recommandée afin de relever le niveau d'énergie envoyée dans la lampe flash. Cette opération nécessite l'intervention d'un opérateur spécialisé.

Dans d'autres appareils un joule mètre (d'un coût prohibitif) est intégré dans la machine afin de garantir la calibration des énergies.

Lors du remplacement d'une pièce à main par une autre, l'énergie envoyée par le générateur peut s'avérer inadaptée à l'état de la lampe flash de la nouvelle pièce à main ou à ses caractéristiques optiques.

La demande US 2005/137 655 décrit une pièce à main pour un appareil de traitement par émission de flashs lumineux, comportant une mémoire non volatile qui contient un identifiant et un historique d'utilisation ainsi que des données de calibration et d'initialisation. Les données de calibration peuvent comporter des données provenant de capteurs de la pièce à main ayant détecté l'énergie émise par la lampe.

Cette publication enseigne de comptabiliser le nombre de flashs pour faciliter les opérations de maintenance, mais est silencieuse sur la possibilité de prendre en considération l'historique de la lampe au cours du fonctionnement de l'appareil afin de tenir compte du vieillissement de la lampe pour déterminer l'énergie à envoyer dans celle-ci.

L'invention vise à améliorer encore les appareils de traitement par émission de flashs lumineux.

La présente invention a ainsi pour objet, selon l'un de ses aspects, un appareil de traitement selon la revendication 1

Tout d'abord, l'énergie envoyée par le générateur peut être adaptée à l'état réel de la lampe et/ou aux caractéristiques optiques de la pièce à main, grâce à des informations transmises par la pièce à main au poste de base.

L'invention peut permettre, si on le souhaite, d'éviter la présence dans la pièce à main d'un capteur recevant la lumière émise par la lampe pour connaître les performances de celle-ci au cours du temps, car ces performances peuvent être estimées à partir de la connaissance du stress effectivement subi par la lampe.

De plus, l'invention peut rendre possible d'indiquer l'état de la lampe flash dès qu'une nouvelle pièce à main est branchée au poste de base.

Enfin, l'intervention d'un opérateur de maintenance n'est plus nécessaire pour relever l'énergie envoyée par le générateur à la lampe flash afin de compenser son vieillissement et le remplacement d'une pièce à main peut être effectué plus facilement, sans risque d'une inadéquation entre l'énergie fournie par le générateur et le résultat recherché.

La mémoire peut contenir une information sur la compensation à apporter à l'énergie envoyée par le générateur en fonction du stress subi. En variante, cette information est contenue dans le poste de base ou déterminée par celui-ci.

La mémoire peut contenir pour chaque traitement le nombre de flashs émis, et le poste de base ou la pièce à main peut répertorier pour chaque traitement le stress subi. Ainsi, connaissant le nombre de flashs émis pour chaque traitement, le stress subi, encore appelé stress cumulé, peut être calculé.

Connaissant ce stress cumulé, l'énergie envoyée par le générateur à la lampe flash à chaque nouveau flash peut être compensée de manière relativement précise, en tenant compte non seulement du nombre de flashs précédemment émis par la lampe mais également du stress réellement subi par celle-ci.

La mémoire peut également contenir d'autres informations, telles que par exemple un identifiant de la pièce à main, qui lui est propre, par exemple un numéro de série.

L'unité de commande et la pièce à main sont agencées pour mémoriser dans la mémoire de la pièce à main au moins une information représentative du stress subi par la lampe flash et éventuellement par d'autres composants optiques au fur et à mesure de l'émission de flashs. Cette mémorisation peut avoir lieu à chaque émission d'un flash.

L'unité de commande est avantageusement agencée pour commander l'énergie envoyée par le générateur vers la pièce à main en fonction de l'état de la lampe flash et du traitement sélectionné, cet état étant déterminé grâce à au moins une information mémorisée dans la pièce à main. L'énergie est ainsi par exemple compensée en fonction de la valeur de stress cumulé actuelle, la valeur de stress cumulé maximale correspondant à la fin de vie de la lampe flash.

Le poste de base est avantageusement agencé pour afficher une information représentative de la durée de vie restante de la pièce à main à partir d'au moins une information contenue dans la mémoire de la pièce à main, notamment du stress cumulé, ce qui peut être utile pour l'utilisateur.

Selon un autre aspect de l'invention, l'unité de commande peut être agencée pour communiquer par un réseau, notamment téléphonique, avec un serveur distant.

L'unité de commande peut être agencée pour interdire le fonctionnement du poste de base en l'absence d'une autorisation, reçue par exemple du serveur distant ou entrée grâce à une interface utilisateur.

L'unité de commande peut comporter une horloge interne et être agencée pour interdire le fonctionnement du poste de base en cas d'absence d'acquisition de l'autorisation dans une période prédéfinie.

L'invention a ainsi encore pour objet, indépendamment de ce qui précède, un appareil de traitement par émission de flashs lumineux, comportant une unité de commande agencée pour n'autoriser le fonctionnement de l'appareil qu'en cas de réception d'une autorisation d'utilisation, cette autorisation étant par exemple transmise par un serveur distant.

L'autorisation est par exemple nécessaire pour permettre le fonctionnement de l'appareil pendant une période prédéfinie ou pour un nombre prédéterminé de flashs.

L'invention a encore pour objet, indépendamment ou en combinaison avec ce qui précède, un appareil de traitement par émission de flashs, comportant :
- une pièce à main comportant la lampe flash et une mémoire permettant de stocker des informations relatives à l'historique de fonctionnement de la lampe,
- un poste de base agencé pour lire la mémoire de la pièce à main, comportant des moyens pour indiquer la durée de vie restante de la pièce à main en fonction d'au moins une information contenue dans la mémoire de la pièce à main.

L'invention pourra être mieux comprise à la lecture de la description détaillée qui va suivre, d'un exemple de mise en oeuvre de celle-ci, et à l'examen du dessin annexé, sur lequel :
- la figure 1 représente de manière schématique, en élévation, un exemple d'appareil conforme à l'invention,
- la figure 2 est un schéma en blocs de l'appareil de la figure 1,
- la figure 3 est un schéma en blocs de la pièce à main,
- la figure 4 illustre différentes étapes de fonctionnement de l'appareil, conformément à un exemple de mise en oeuvre de l'invention,
- la figure 5 illustre l'évolution du facteur de compensation en fonction de la valeur de stress cumulé, et
- la figure 6 est une table donnant des valeurs d'incrément de la valeur de stress cumulé en fonction du type de traitement effectué.

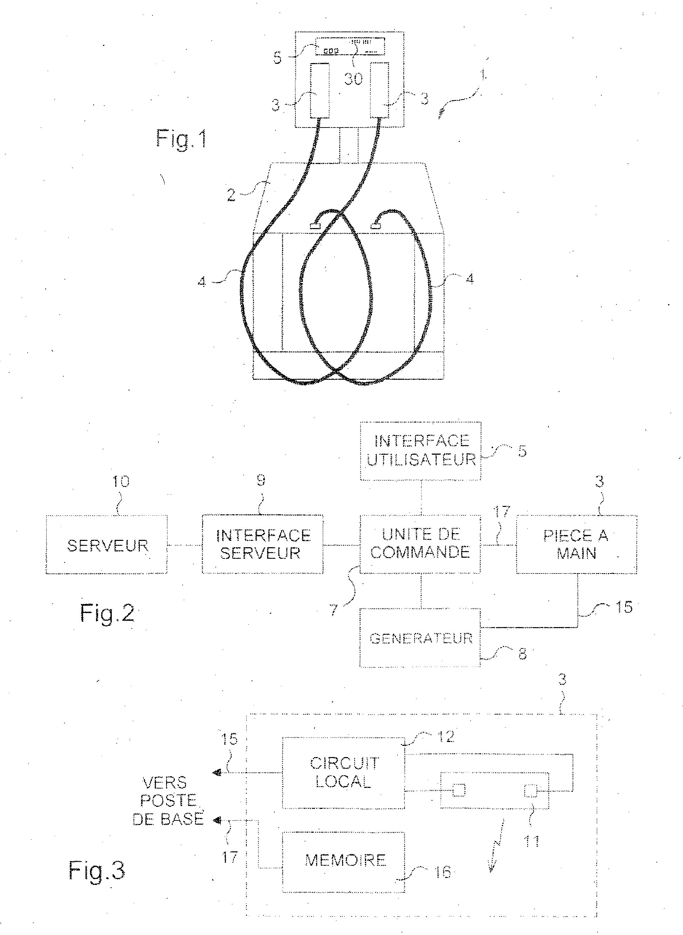

On a représenté à la figure 1 un appareil 1 de traitement par émission de flashs lumineux, comportant un poste de base 2 et au moins une pièce à main 3 reliée par un câble 4 au poste de base 2.

Dans l'exemple considéré, l'appareil 1 comporte deux pièces à main 3 reliées chacune au poste de base, ces pièces à main 3 présentant par exemple des caractéristiques optiques différentes, l'une étant par exemple destinée à l'épilation et l'autre à un traitement vasculaire ou de photorajeunissement.

Le poste de base 2 comporte une interface utilisateur 5 reliée à une unité de commande 7 comportant par exemple un ou plusieurs microprocesseurs ou microcontrôleurs.

L'interface utilisateur 5 permet par exemple la sélection d'un traitement et l'indication du phototype pour l'épilation notamment.

L'unité de commande 7 échange des informations avec l'interface utilisateur 5 et contrôle également le fonctionnement d'un générateur haute tension 8 qui est par exemple conforme à celui décrit dans la demande de brevet français FR 2 838 042. L'unité de commande 7 contrôle notamment l'énergie envoyée dans la lampe flash en fonction du traitement sélectionné et des informations stockées dans la pièce à main, comme détaillé par la suite.

L'unité de commande 7 peut également échanger des informations avec une interface serveur 9 pouvant communiquer par une liaison filaire ou non avec un serveur distant 10, comme cela sera précisé dans la suite.

La pièce à main 3 comporte au moins une lampe flash 11, comme illustré à la figure 3, laquelle peut être reliée par l'intermédiaire d'un circuit électrique local 12, intégré dans la pièce à main 3, au générateur 8.

Le circuit électrique local 12 est relié par une liaison électrique 15 intégrée dans le câble 4 au générateur 8 et comporte un interrupteur non apparent, permettant de commander l'émission d'un flash lumineux lorsqu'actionné par l'utilisateur.

Conformément à un aspect de l'invention, la pièce à main 3 comporte au moins une mémoire électronique 16 pouvant échanger des informations par une liaison électrique 17 avec l'unité de commande 7.

La mémoire 16 est par exemple contenue dans un microcontrôleur, par exemple celui commercialisé sous la dénomination commerciale PICxxx par la société Microchip.

L'échange des informations entre la pièce à main 3 et l'unité de commande 7 s'effectue par exemple selon le protocole I²C.

La mémoire 16 permet d'enregistrer dans la pièce à main 3, préalablement à sa commercialisation, des informations concernant le stress subi par la lampe flash à chaque flash en fonction de l'énergie reçue, la valeur de stress cumulé maximale en fm de vie et éventuellement l'évolution du facteur de compensation à apporter à l'énergie envoyée vers la lampe flash en fonction de la valeur de stress cumulé.

La mémoire 16 permet également d'enregistrer des informations concernant l'historique d'utilisation de la lampe flash.

La mémoire 16 peut également comporter des informations sur des caractéristiques électriques et/ou optiques de la pièce à main, notamment son rendement, et un identifiant de la pièce à main, par exemple un numéro de série.

La mémoire peut mémoriser autant de variables de comptage que de traitements pouvant être effectués par le générateur, chaque traitement correspondant à des caractéristiques particulières du flash (énergie, durée, ...) et engendrant sur la lampe un stress correspondant.

Chaque valeur de comptage peut être incrémentée d'une unité lors de l'émission du flash correspondant.

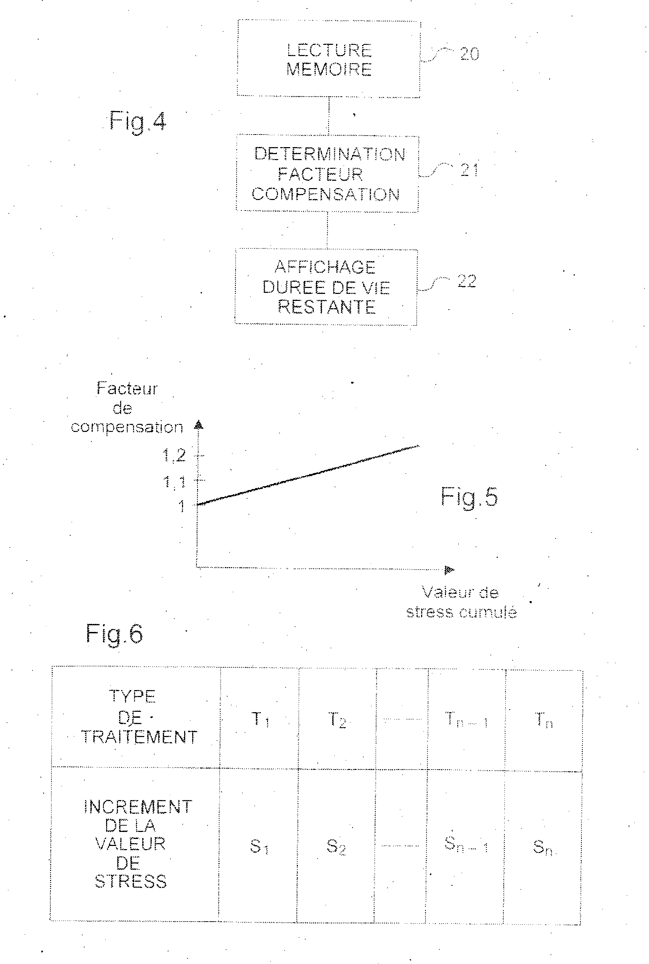

On a représenté à la figure 6 une table pouvant être mémorisée dans la mémoire 16, comportant par exemple des incréments S₁ à Sₙ représentatifs du stress subi par la lampe flash lors d'un flash en fonction de traitement respectifs T₁ à Tₙ correspondant à des énergies différentes envoyées dans la lampe flash.

T₁ à Tₙ₋₁ concernent par exemple des traitements d'épilation pour des phototypes différents et Tₙ un traitement d'élimination de vaisseaux sanguins. Les énergies correspondant à ces traitements sont différentes et font subir à la lampe flash un stress qui n'est pas le même.

Bien entendu, le nombre de traitements possibles correspondant à des énergies différentes peut différer d'un appareil à l'autre. Des énergies, courants, durées de flash et/ou tensions peuvent encore être mémorisés dans la mémoire plutôt que des natures de traitements.

Lors de la connexion d'une pièce à main 3 au poste de base 2, l'unité de commande 7 lit la mémoire 16 dans une première étape 20, comme illustré à la figure 4.

L'unité de commande 7 est alors renseignée sur le stress subi par la lampe en fonction d'un traitement, à chaque flash, grâce à la lecture des incréments S₁ à Sₙ.

Le stress subi par la lampe peut être déterminé à partir de la connaissance du nombre de flashs émis pour chaque traitement et du stress lié à chaque traitement, en additionnant l'ensemble.

Une valeur de stress cumulé peut encore être contenue dans la mémoire et l'unité de commande 7 peut lire cette valeur de stress cumulé, représentative du stress subi par la lampe depuis le début de son fonctionnement.

L'unité de commande 7 peut déterminer à l'étape 21, en fonction du stress subi, le facteur de compensation à apporter à l'énergie délivrée à la lampe flash.

L'unité de commande 7 ou la mémoire 16 peuvent à cet effet contenir une information concernant un facteur de compensation à apporter en fonction de la valeur de stress cumulé. Ce facteur de compensation est par exemple donné par la pente d'une droite décrivant l'évolution du facteur de compensation en fonction d'une valeur de stress cumulé, comme illustré à la figure 5.

Lorsque l'utilisateur commande l'émission d'un flash, l'énergie envoyée à la lampe flash tient compte du facteur de compensation précédemment déterminé, qui est par exemple de 1,2 en fin de vie de la lampe. Cela signifie que 20 % d'énergie en plus qu'en début de vie de la lampe doit être envoyée à celle-ci pour produire une même densité surfacique d'énergie sur la peau.

A chaque émission de flash, le stress subi peut être actualisé en étant incrémentée de la valeur Sᵢ correspondant au traitement Tᵢ effectué.

Lorsque la pièce à main 3 est séparée du poste de base 3, les informations contenues dans la mémoire 16 y restent contenues.

Ainsi, en cas de changement de pièce à main, le poste de base 2 dispose des informations nécessaires pour que l'énergie envoyée par le générateur 8 à la nouvelle pièce à main permette d'atteindre le résultat recherché.

Le poste de base 2 peut également afficher, par exemple grâce à un bar graph et/ou un afficheur 30 de l'interface utilisateur 5, l'état de la lampe flash de la pièce à main 3 utilisée, ce qui correspond à l'étape 22 de la figure 4.

Par exemple, si le stress subi, découlant des informations mémorisées dans la pièce à main, est à la moitié de la valeur maximale correspondant à la fin de vie, qui peut également être mémorisée dans la pièce à main et qui dépend par exemple des caractéristiques de la lampe flash, l'utilisateur sait grâce au bar graph 30 que la lampe flash est à la moitié de sa vie.

Ainsi, l'utilisateur peut être prévenu assez tôt d'un changement de pièce à main à prévoir.

Le cas échéant, l'unité de commande 7 peut interdire l'émission d'un flash si la lampe flash est en fin de vie, ce qui accroît la sécurité.

Selon un autre aspect de l'invention, l'unité de commande 7 peut requérir une autorisation pour permettre le fonctionnement de l'appareil 1.

Cette autorisation est par exemple un code entré sur l'interface utilisateur 5 ou une information obtenue par connexion au serveur distant 10.

Cela peut permettre par exemple à une société de louer l'appareil 1 à des utilisateurs sans craindre que ceux-ci exploitent sans payer l'appareil, puisqu'à défaut d'une autorisation reçue du serveur distant 10, le fonctionnement de l'appareil est empêché.

L'interrogation du serveur distant 10 afin de recueillir l'autorisation peut par exemple s'effectuer de manière périodique et automatique grâce à une horloge interne de l'unité de commande 7. Par exemple, tous les mois, l'unité de commande 7 peut interroger le serveur distant 10 et, à défaut d'avoir reçu l'autorisation, le fonctionnement est empêché.

L'autorisation reçue peut avoir une durée de validité limitée, déterminée par exemple grâce à une horloge interne de l'unité de commande 7 ou par un nombre de flashs émis suite à la réception de l'autorisation.

L'unité de commande 7 peut également adresser au serveur distant 10 des informations concernant l'utilisation de l'appareil et notamment lui transmettre les identifiants des pièces à main utilisées et le nombre de flashs émis par chacune d'elles, voire les valeurs de stress cumulé.

Cela peut permettre par exemple au fabricant de l'appareil de fournir à l'utilisateur une nouvelle pièce à main lorsque la lampe flash de la pièce à main utilisée sur l'appareil est proche de sa fin de vie. Le délai d'attente d'une nouvelle pièce à main peut ainsi être réduit.

Bien entendu, l'invention n'est pas limitée à l'exemple qui vient d'être décrit.

L'appareil 1 peut notamment ne pas comporter d'interface serveur 9 et fonctionner au moyen d'une carte à puce comportant un compteur décrémenté à chaque flash.

## Revendications

1. Appareil (1) de traitement par émission de flashs lumineux, comportant une pièce à main (3) comportant au moins une lampe flash (11) reliée par un câble (4) à un poste de base (2) comportant un générateur électrique (8) pouvant effectuer plusieurs traitements différents, chaque traitement correspondant à des caractéristiques particulières du flash et engendrant sur la lampe un stress correspondant,
la pièce à main (3) comportant au moins une mémoire (16) permettant d'enregistrer des informations relatives à l'historique de son fonctionnement, **caractérisé par le fait que** la mémoire (16) étant configurée pour mémoriser autant de variables de comptage que de traitements pouvant être effectués par le générateur (8), chaque traitement correspondant à des caractéristiques particulières choisies parmi la tension, le courant ou la durée d'un flash, et en ce que le poste de base (2) comporte au moins une unité de commande (7) agencée pour échanger des données avec la pièce à main (3), afin de permettre au poste de base de déterminer le stress subi par la lampe durant son fonctionnement passé, chaque variable de comptage étant incrémentée d'une unité lors de l'émission du flash correspondant.

2. Appareil selon la revendication 1, dans lequel l'unité de commande (7) et la pièce à main (3) sont agencées pour mémoriser dans la mémoire (16) de la pièce à main au moins une valeur de stress cumulé représentative du stress subi par la lampe flash.

3. Appareil selon la revendication 2, dans lequel l'unité de commande (7) est agencée pour contrôler l'énergie envoyée par le générateur vers la pièce à main en fonction d'au moins une information mémorisée dans la pièce à main.

4. Appareil selon l'une quelconque des revendications 1 à 3, dans lequel le poste de base (2) est agencé pour afficher une information représentative de la durée de vie restante de la pièce à main, à partir d'au moins une information contenue dans la mémoire (16) de la pièce à main.

5. Appareil selon l'une quelconque des revendications 1 à 4, agencé pour que la mémoire (16) de.la pièce à main comporte au moins une information sur son rendement.

6. Appareil selon l'une quelconque des revendications 1 à 5, agencé pour que la mémoire (16) de la pièce à main comporte des incréments (S₁, S₂, ... Sₙ) représentatif du stress généré par différents traitements (T₁, T₂, ..., Tₙ).

7. Appareil selon l'une quelconque des revendications 1 à 6, dans lequel la mémoire contient une information sur le nombre de flashs émis pour chaque traitement.

8. Appareil selon la revendication 7, dans lequel le poste de base est agencé pour calculer le stress subi par la lampe à partir du nombre de flashs émis pour chaque traitement.

9. Appareil selon l'une quelconque des revendications 1 à 8, dans lequel la mémoire (16) de la pièce à main est contenue dans un microcontrôleur.

10. Appareil selon l'une quelconque des revendications 1 à 9, la mémoire (16) de la pièce à main contenant une information sur un facteur de compensation à apporter à l'énergie envoyée par le générateur (8) à la lampe flash (11) en fonction d'une valeur de stress cumulé.

11. Appareil selon l'une quelconque des revendications 1 à 10, la mémoire (16) de la pièce à main contenant une information sur une valeur de stress cumulé maximale correspondant à la fin de vie de la lampe.

12. Appareil selon l'une quelconque des revendications 1 à 11, dans lequel l'unité de commande (7) est agencée pour communiquer par un réseau avec un serveur distant (10).

13. Appareil selon la revendication 12, dans lequel l'unité de commande (7) est agencée pour interdire le fonctionnement du poste de base en l'absence d'une autorisation reçue du serveur (10).

14. Appareil selon l'une quelconque des revendications 1 à 13, dans lequel l'unité de commande (7) comporte une horloge interne et dans lequel l'unité de commande est agencée pour interdire le fonctionnement du poste de base en cas d'absence d'acquisition d'une autorisation dans une période prédéfinie.

## Claims

1. Apparatus (1) for treatment with light flashes, with a handpiece (3) comprising at least one flash lamp (11) linked by a cable (4) to a base station (2) comprising an generator (8) which can perform several different treatments, each treatment corresponding to particular flash characteristics and causing a corresponding stress on the lamp, the handpiece (3) comprising at least one memory (16) allowing recording of information relating to its operating history, **characterised in that** the memory (16) is configured to memorise as many counting variables as treatments which can be performed by the generator (8), each treatment corresponding to to particular selected characteristics including voltage, current or duration of a flash, and that the base station (2) comprises at least one control unit (7) adapted to exchange data the handpiece (3) in order to allow the base station to determine the stress caused to the lamp during its previous operation, each counting variable being incremented by one unit on emission of the corresponding flash.

2. Apparatus according to claim 1, wherein the control unit (7) and the handpiece (3) are adapted to memorise in the handpiece memory (16) at least one cumulated stress value representative of the stress caused to the flash lamp.

3. Apparatus according to claim 2, wherein the control unit (7) is adapted to control the energy supplied by the generator to the handpiece as a function of at least one datum memorised in the handpiece.

4. Apparatus according to any of claims 1 to 3, wherein the base station (2) is adapted to display information representative of the remaining life of the handpiece, based on at least one datum contained in the handpiece memory (16).

5. Apparatus according to any of claims 1 to 4, adapted such that the handpiece memory (16) comprises at least one datum on its output.

6. Apparatus according to any of claims 1 to 5, adapted such that the handpiece memory (16) comprises increments (S₁, S₂,... Sₙ) representative of the stress by different treatments (T₁, T₂, ... Tₙ).

7. Apparatus according to any of claims 1 to 6, wherein the memory contains information on the number of flashes emitted for each treatment.

8. Apparatus according to claim 7, wherein the base station is adapted to calculate the stress to the lamp from the number of flashes emitted for each treatment.

9. Apparatus according to any of claims 1 to 8, wherein the handpiece memory (16) is contained in a microcontroller.

10. Apparatus according to any of claims 1 to 9, wherein the handpiece memory (16) contains information on a compensation factor to be applied to the energy emitted by the (8) to the lamp (11) as a function of a cumulated stress value.

11. Apparatus according to any of claims 1 to 10, wherein the handpiece memory (16) contains information on a maximum cumulated stress value corresponding to the end of life of the lamp.

12. Apparatus according to any of claims 1 to 11, wherein the control unit (7) is adapted to with a remote server (10) via a network.

13. Apparatus according to claim 12, wherein the control unit (7) is adapted to prohibit the operation of the base station in the absence of authorisation received the server (10).

14. Apparatus according to any of claims 1 to 13, wherein the control unit (7) comprises an internal clock and wherein the control unit is adapted to prohibit the function of the base station unless authorization is received within a predefined period.

## Patentansprüche

1. Vorrichtung (1) zur Verarbeitung durch Emission von Lichtblitzen, die ein Handstück (3) umfasst, das mindestens eine Blitzlampe (11) umfasst, die durch ein Kabel (4) mit einer Basisstation (2) verbunden ist, die einen Stromgenerator (8) umfasst, der mehrere verschiedene Verarbeitungen durchführen kann, wobei jede Verarbeitung bestimmten Eigenschaften des Blitzes entspricht und auf der Lampe eine entsprechende Belastung erzeugt,
wobei das Handstück (3) mindestens einen Speicher (16) umfasst, der das Speichern von Informationen ermöglicht, die den Verlauf seines Betriebs betreffen, **dadurch gekennzeichnet, dass** der Speicher (16) konfiguriert ist, um so viele Zählungsvariablen zu speichern, wie Verarbeitungen durch den Generator (8) durchgeführt werden können, wobei jede Verarbeitung bestimmten unter der Spannung, dem Strom oder der Dauer eines Blitzes gewählten Eigenschaften entspricht, und dadurch, dass die Basisstation (2) mindestens eine Steuereinheit (7) umfasst, die gestaltet ist, um Daten mit dem Handstück (3) auszutauschen, um der Basisstation das Bestimmen der durch die Lampe während ihres vergangenen Betriebs erlittenen Belastung zu ermöglichen, wobei jede Zählungsvariable bei der Emission des entsprechenden Blitzes um eine Einheit erhöht wird.

2. Vorrichtung nach Anspruch 1, wobei die Steuereinheit (7) und das Handstück (3) gestaltet sind, um im Speicher (16) des Handstücks mindestens einen kumulierten Belastungswert zu speichern, der für die durch die Blitzlampe erlittene Belastung charakteristisch ist.

3. Vorrichtung nach Anspruch 2, wobei die Steuereinheit (7) gestaltet ist, um die Energie, die durch den Generator zum Handstück gesendet wird, als Funktion von mindestens einer in dem Handstück gespeicherten Information zu steuern.

4. Vorrichtung nach einem der Ansprüche 1 bis 3, wobei die Basisstation (2) gestaltet ist, um eine Information, die für die restliche Lebensdauer des Handstücks charakteristisch ist, ausgehend von mindestens einer Information anzuzeigen, die im Speicher (16) des Handstücks enthalten ist.

5. Vorrichtung nach einem der Ansprüche 1 bis 4, die gestaltet ist, damit der Speicher (16) des Handstücks mindestens eine Information über seinen Wirkungsgrad umfasst.

6. Vorrichtung nach einem der Ansprüche 1 bis 5, die gestaltet ist, damit der Speicher (16) des Handstücks Inkremente (S₁, S₂, ... Sₙ) umfasst, die für die durch verschiedene Verarbeitungen (T₁, T₂, .... Tₙ) erzeugte Belastung charakteristisch sind.

7. Vorrichtung nach einem der Ansprüche 1 bis 6, wobei der Speicher eine Information über die Anzahl der für jede Verarbeitung emittierten Blitze enthält.

8. Vorrichtung nach Anspruch 7, wobei die Basisstation gestaltet ist, um die durch die Lampe erlittene Belastung ausgehend von der Anzahl der für jede Verarbeitung emittierten Blitze zu berechnen.

9. Vorrichtung nach einem der Ansprüche 1 bis 8, wobei der Speicher (16) des Handstücks in einem Mikrocontroller enthalten ist.

10. Vorrichtung nach einem der Ansprüche 1 bis 9, wobei der Speicher (16) des Handstücks eine Information über einen Ausgleichsfaktor enthält, der als Funktion eines kumulierten Belastungswerts bei der durch den Generator (8) an die Blitzlampe (11) gesendeten Energie einzubringen ist.

11. Vorrichtung nach irgendeinem der Ansprüche 1 bis 10, wobei der Speicher (16) des Handstücks eine Information über einen maximalen kumulierten Belastungswert enthält, der dem Ende der Lebensdauer der Lampe entspricht.

12. Vorrichtung nach einem der Ansprüche 1 bis 11, wobei die Steuereinheit (7) gestaltet ist, um über ein Netz mit einem entfernten Server (10) zu kommunizieren.

13. Vorrichtung nach Anspruch 12, wobei die Steuereinheit (7) gestaltet ist, um den Betrieb der Basisstation beim Nichtvorhandensein einer von dem Server (10) empfangenen Erlaubnis zu sperren.

14. Vorrichtung nach einem der Ansprüche 1 bis 13, wobei die Steuereinheit (7) einen Taktgeber umfasst und wobei die Steuereinheit gestaltet ist, um den Betrieb der Basisstation im Falle des Nichtvorhandenseins der Erfassung einer Erlaubnis innerhalb eines vorbestimmten Zeitraums zu sperren.
